Europäisches Patentamt

European Patent Office

Office européen des brevets

(11) Veröffentlichungsnummer: **0 435 132 B1**

(12) **EUROPÄISCHE PATENTSCHRIFT**

(45) Veröffentlichungstag der Patentschrift: **17.08.94**

(21) Anmeldenummer: **90124557.1**

(22) Anmeldetag: **18.12.90**

(51) Int. Cl.5: **C12N 15/09**, C12P 13/08, C07H 21/04, C12N 1/21, //(C12P13/08,C12R1:15), (C12P13/08,C12R1:13)

(54) **Verfahren zur fermentativen Herstellung von Aminosäuren, insbesondere L-Lysin, dafür geeignete Mikroorganismen und rekombinante DNA.**

(30) Priorität: **27.12.89 DE 3943117**

(43) Veröffentlichungstag der Anmeldung:
**03.07.91 Patentblatt 91/27**

(45) Bekanntmachung des Hinweises auf die Patenterteilung:
**17.08.94 Patentblatt 94/33**

(84) Benannte Vertragsstaaten:
**BE DE FR GB IT NL SE**

(56) Entgegenhaltungen:
**EP-A- 0 143 195
EP-A- 0 197 335
EP-A- 0 219 027
EP-A- 0 387 527
DE-A- 3 823 451**

(73) Patentinhaber: **Forschungszentrum Jülich GmbH
Wilhelm-Johnen-Strasse
D-52425 Jülich (DE)**

(72) Erfinder: **Cremer, Josef, Dr.
Höhenstrasse 65
W-5165 Hürtgenwald 2 (DE)**
Erfinder: **Eggeling, Lothar, Dr.
Elsenkamp 6
W-5170 Jülich (DE)**
Erfinder: **Sahm, Hermann, Prof.
Wendelinusstrasse 71
W-5170 Jülich (DE)**

Rank Xerox (UK) Business Services
(3.10/3.09/3.3.3)

**Beschreibung**

Die Erfindung bezieht sich auf ein Verfahren zur Herstellung von Aminosäure, insbesondere von L-Lysin, durch Züchten eines Mikroorganismus der Gattung Corynebacterium oder Brevibacterium, der eine rekombinante DNA aus Vektor-DNA und für die Produktion von Proteinen mit dapA-Aktivität kodierende DNA-Sequenz enthält, in einem Nährmedium und Gewinnung der Aminosäure aus dem Kulturmedium, und sie umfaßt dafür geeignete Mikroorganismen und rekombinante DNA.

Corynebacterium glutamicum und verwandte Gattungen wie z. B. Brevibacterium lactofermentum und Brevibacterium flavum sind als Aminosäuren bildende Mikroorganismen bekannt. Um deren Produktivität zu erhöhen, wurden ungerichtete Mutationen mit physikalischen und chemischen Mitteln durchgeführt.

Neben dieser klassischen Methode zur Produktionssteigerung wurden auch bereits Vektorsysteme entwickelt, die die Anwendung rekombinanter DNA-Techniken für Mikroorganismen der Gattungen Corynebacterium und Brevibacterium ermöglichen und mit denen durch Amplifikation von Biosynthesegenen die Ausscheidungsmenge von Aminosäuren erhöht werden kann.

So ist aus der EP-A2-0 219 027 die Klonierung eines DNA-Fragmentes aus Corynebacterium bekannt, das einen Escherichia coli Stamm mit defektem Gen für die Aspartatsemialdehyddehydrogenase (asd) oder die Aspartat-Aminotransferase (AAT) komplementiert. Das klonierte Fragment führt nach Transformation eines Lysin ausscheidenen Corynebacterium Stammes zu gesteigerter Lysinbildung.

In der EP-A1-0 143 195 wird die Klonierung eines DNA-Fragmentes und dessen positive Auswirkung auf die Lysinbildung beschrieben, das Phosphoenolpyruvatcarboxylase defekte Mutanten komplementiert.

Als ein weiteres DNA-Fragment wird in der EP-A1-0 197 335 ein die Dihydrodipicolinatsynthase (dapA) komplementierendes Gen angegeben, das ebenfalls zu gesteigerter Lysinbildung führt und ggf. zusammen mit für Tetrahydropicolinsäure-Succinylase kodierendem Gen vorgesehen wird.

In der De-Patentanmeldung P 39 08 201.6 wird ein Verfahren zur fermentativen Herstellung von L-Lysin beschrieben, bei dem eine in Corynebacterium oder Brevibacterium replizierende rekombinante DNA aus Vector-DNA und für asd und/oder LysC (deregulierte Aspartatkinase) kodierenden DNA-Sequenzen für die Transformation der Mikroorganismen vorgesehen wird.

In der DE-Patentanmeldung P 38 23 451.3 wird schließlich eine in Corynebacterium oder Brevibacterium replizierbare rekombinante DNA aus Vektor-DNA und asd Gen und/oder dapA Gen angegeben, die zur Transformation von Mikroorganismen zur Gewinnung von Aminosäuren der Aspartat-Familie, insbesondere von L-Lysin dienen soll.

Diese unterschiedlichen gentechnologischen Verfahren haben bislang noch nicht zu einer allseits akzeptierten Aminsäureproduktion geführt.

Ziel der Erfindung ist daher ein Verfahren zur fermentativen Aminosäureproduktion, das zu einer verbesserten Aminosäure-, insbesondere L-Lysinausscheidungsrate führt.

Das zu diesem Zweck entwickelte erfindungsgemäße Verfahren der eingangs genannten Art ist dadurch gekennzeichnet, daß die rekombinante DNA zusätzlich eine für die Produktion von Proteinen mit LysC-Aktivität kodierende DNA-Sequenz enthält.

Weitere Besonderheiten der Erfindung gehen aus den Patentansprüchen hervor.

Als Donorstämme können alle (bevorzugt L-Lysin produzierenden) Bakterien der Gattung Brevibacterium und Corynebacterium dienen, die die entsprechenden Gene enthalten, insbesondere aber Corynebacterium glutamicum MH-20, das durch Mutagenese von Corynebacterium ATCC 13032 mit Nitronitrosoguanidin entwickelt wurde, und deregulierte Aspartatkinase (LysC) enthält. Dieser Stamm ist unter der Nummer DSM 5714 hinterlegt.

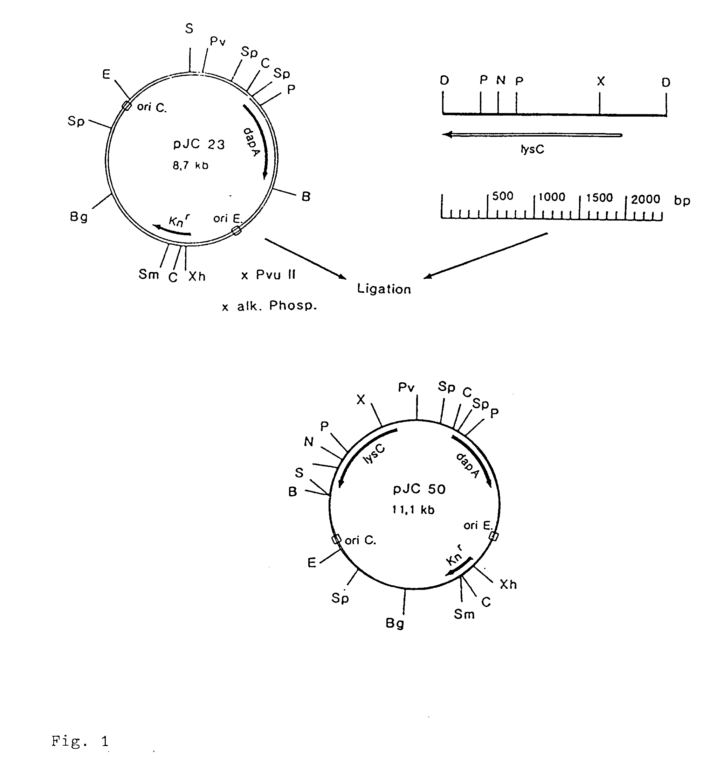

Die chromosomale DNA wird aus dem Donor auf bekannte Weise extrahiert und mit Restriktionsendonukleasen behandelt. Nach der Konstruktion der rekombinanten DNA durch Einführung chromosomaler DNA-Fragmente in einen Vektor erfolgt die Transformation des Mikroorganismus mit dem so gewonnenen Plasmid, erfindungsgemäß beispielsweise mit pJC50, dessen Restrikionskarte in Figur 1 dargestellt ist, und das in dem Stamm Corynebacterium glutamicum MH 20-22B unter der Nummer DSM 5715 bei der Deutschen Sammlung für Mikroorganismen und Zellkulturen gemäß dem Budapester Abkommen hinterlegt wurde.

Ein bevorzugtes Vektorsystem stellt pZ1 dar (EP-A1-0318663) oder dessen Derivat pJC1 (Cremer et al., J. General Microbiol. 134 (1988) 3221-3229). Verwendbar sind aber auch die aus der EP-A-93 611 bekannten zusammengesetzten Plasmide, soweit sie in Corynebakterien oder Brevibakterien replizieren, insbesondere pAJ655, pAJ611, pAJ440, pAJ1844 und pAJ3148, aber auch pCG11 oder pCE54 (EP-A 0 233 581).

Das konstruierte Plasmid enthält die Kombination der klonierten Gene dapA und lysC. Transformanten, die dieses Plasmid aufweisen, enthalten eine bezüglich der feed back Inhibition durch L-Threonin und L-

EP 0 435 132 B1

Lysin erhöhte deregulierte Aspartatkinase Aktivität, sowie gleichzeitig erhöhte Dihydrodipicolinatsynthase Aktivität. Enzymmessungen haben ergeben, daß die Aktivität der Asparat Kinase in solchen Transformanten etwa 15 fach und die der Dihydrodipicolinat Synthase ebenfalls etwa 15 fach erhöht ist.

Die bei dieser Kombination der Gene erreichten Enzymaktivitäten gewährleisten sowohl eine effiziente Einschleusung von Aspartat in die Lysinbiosynthesesequenz (durch die deregulierte erhöhte Aspartatkinase Aktivität), als auch eine Überwindung der in der Dihydrodipicolinatsynthase bestehenden Limitation innerhalb der Biosynthesesequenz.

Es ergibt sich somit eine wesentliche Verbesserung dadurch, daß gleichzeitig die den Subtratfluß kontrollierenden Enzyme der Lysinbiosynthesesequenz überexprimiert werden, wobei durch die Kombination eine L-Lysinausscheidung bewirkt oder eine vorhandene verbessert wird.

Die Herstellung von L-Lysin produzierenden Stämmen, die bevorzugt angewandt werden, ist in der US-PS 4 275 157 sowie im J. Gen. Appl. Microbiol. 15 (1969) 267-87 beschrieben.

Die Erzeugung von Mutanten mit verminderter Feed back Hemmung der Aspartatkinase kann, wie im J. Biochem. (Tokyo) Bd. 68 (1970) Seiten 701-710 oder in Agr. Biol. Chem. (Tokyo) 48 (1984) Seiten 3091-3098 beschrieben, erfolgen.

Nachfolgend wird die Erfindung anhand von Beispielen näher beschrieben:

Beispiel 1: Herstellung rekombinanter Stämme

A) Klonierung eines DNA-Fragmentes von Corynebacterium glutamicum, das für Dihydrodipicolinatsynthaseaktivität kodiert (dapA).

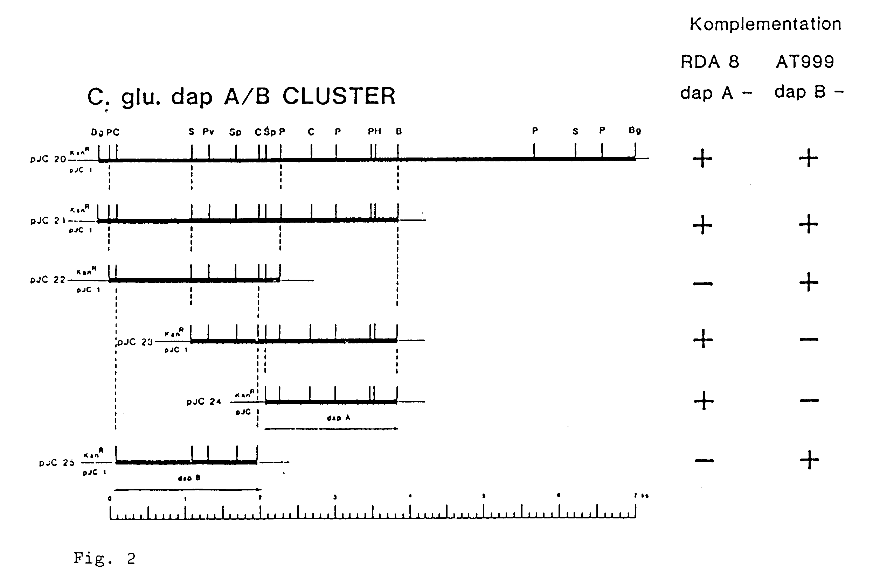

Gesamt-DNA wurde aus C. glutamicum ATCC 13032 - wie bei Chater et al. ( Curr. Topics Microb. Immunol. 96 (1982) 69) beschrieben - isoliert und partiell mit dem Restriktionsenzym Sau3A verdaut. Mit der DNA wurden Cosmide in Escherichia coli ED8767 mit dem Cosmidvektor pHC79 entsprechend der Vorschrift des Herstellers (Boehringer Mannheim, 6800 Mannheim 31) konstruiert. Diese Cosmide wurden wie bei Maniatis et al. beschrieben isoliert (T. Maniatis et al., "Molecular Cloning", A Laboratory Manual (1982) Cold Spring Harbour), und benutzt, den im Dihydrodipicolinatsynthasegen dapA defekten E. coli Stamm RDA8 zu komplementieren. Ein Cosmid wurde isoliert und durch Subklonierung in E. coli RDA8 nach den bekannten Verfahren ein 2,7 kb Sal1/BamH1 DNA Fragment isoliert. Dieses Fragment wurde in die multiple cloning site von pJC1 ligiert und so das Plasmid pJC23 (Figur 2) erhalten. Dieses in E. coli konstruierte Plasmid wurde durch Sphäroplastentransformation (Thierbach et al., Appl Microbiol Biot 29 - (1988) 356) nach C. glutamicum transferiert

B) Klonierung eines DNA Fragmentes, das für deregulierte Aspartatkinase Aktivität kodiert.

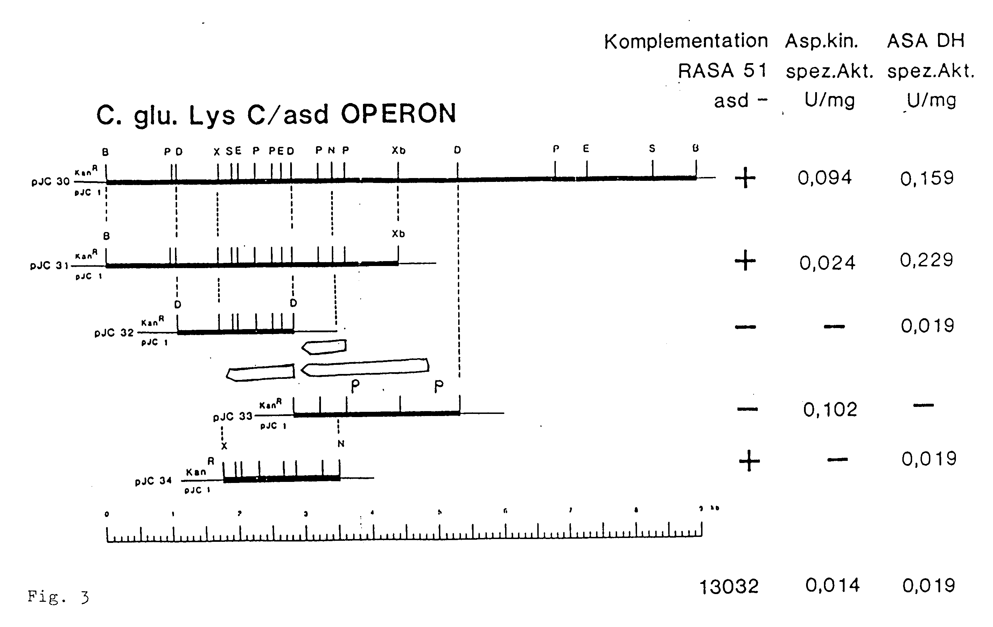

Chromosomale DNA wurde aus C. glutamicum MH 20 isoliert und Cosmide wie in Beispiel 1 beschrieben in E. coli ED8767 hergestellt. Der Stamm MH 20 ist vom Wildtyp C. glutamicum ATCC13032 abgeleitet und zeichnet sich dadurch aus, daß dessen Aspartatkinase insensitiv gegenüber der feed back Inhibierung durch L-Lysin plus L-Threonin ist (Cremer et al., J General Microbiology 134 (1988) 3221). Cosmide wurden isoliert und der im Aspartatsemialdehyddehydrogenasegen asd defekte E. coli Stamm RASA51 damit transformiert. Das Transformationsgemisch wurde auf LB-Agar mit 50μg/ml Ampicillin ausplattiert und die Platten bei 30°C inkubiert. Plasmid DNA aus emporgewachsenen Kolonien wurde isoliert, mit dem Restriktionsenzym BamH1 gespalten, und mit dem ebenfalls BamH1 gespaltenen und mit alkalischer Phosphatase behandeltem Vektor pJC1 ligiert. Mit dem Ligationsansatz wurde wiederum der E. coli Stamm RASA51 transformiert und auf LB-Agar ausplattiert. Aus einzelnen Kolonien wurde Plasmid DNA präpariert und das Plasmid pJC30 erhalten. Dieses Plasmid wurde durch Restriktionskartierung weiter kartiert. Es enhält eine etwa 8,9 kb lange Insertion chromosomaler DNA. Die Restriktionskarte ist in Figur 3 dargestellt. Von pJC30 wurden verschiedene Subklone in pJC1 hergestellt, mit denen der Wildtyp von C. glutamicum transformiert wurde. Von Transformanten wurden Messungen der Aspartatkinase und Aspartatsemialdehyddehydrogenase Aktivität durchgeführt (Figur 3), die zeigen, daß pJC33 die genetische Information für das Aspartatkinaseenzym enthält.

C) Konstruktion eines dapA und lysC enthaltenden Vektors pJC50.

Aus dem Vektor pJC30 (gemäß B) wurde das für die deregulierte Aspartatkinase kodierende DNA-Fragment isoliert. Dazu wurde der Vektor mit dem Restriktionsenzym Dra1 verdaut und die resultierenden Fragmente in einem Agarosegel aufgetrennt. Das für die Aspartatkinase kodierende, etwa 2,5 kb große DNA-Fragment wurde aus dem Gel wie bei Maniatis et al. beschrieben isoliert und aufgereinigt. Als Vektor wurde pJC23 (gemäß A) benutzt, der bereits das Gen für die Dihydrodipicolinatsynthase trägt. Dieser Vektor wurde mit Pvu2 linearisiert, zur Entfernung der entständigen Phosphatgruppen mit alkalischer Phosphatase behandelt, mit dem isolierten Dra1 Fragment vermischt, und mit T4-Ligase behandelt. Mit dem Ligationsgemisch erfolgte die Transformation von Escherichia coli RDE51. Transformanten davon wurden auf LB-Kanamycinplatten erhalten und von einigen Plasmide isoliert. Neun von zehn Transformanten enthielten das rekombinante Plasmid pJC50, das gleichzeitig die Gene für deregulierte Aspartatkinase und Dihydrodipicolinatsynthase trägt. Eine Restriktionskarte davon wurde erstellt, und ist in Abbildung 1 wiedergegeben.

D) Überexpression der Aspartatkinase und Dihydrodipicolinatsynthase Aktivität in rekombinanten Corynebacterium glutamicum Stämmen durch pJC50.

Das in E. coli konstruierte Plasmid pJC50 wurde mittels Sphäroplastentransformation in den Wildtyp von Corynebacterium glutamicum und dessen Derivat DG52-5, das sich durch deregulierte Aspartatkinase Aktivität auszeichnet transformiert. Der Stamm DG-52-5 ist bei Cremer et al. (a. a. 0.) beschrieben.
Die Aspartatkinase Aktivität und Dihydrodipicolinatsynthase Aktivität wurde in den pJC50 enthaltenen Stämmen bestimmt. Als Kontrollen wurden die Aktivitäten zusätzlich in den Ausgangsstämmen und in den pJC23 (nur Dihydrodipicolinatsynthase) und pJC30 (nur Aspartatkinase) enthaltenen Stämmen gemessen. Die Stämme wurden dazu in einem Komplexmedium, das 2,5 g/l NaCl, 10 g/l Pepton, 10 g/l Hefeextrakt sowie 2% Glukose (pH7,4) enthielt über Nacht angezogen. Die Zellen wurden durch Zentrifugation geerntet, in einem geringen Volumen 100 mM Tris-HCl, 50 mM Ammoniumsulfat (pH8) aufgenommen und mit dem Ultraschallgerät Branson Sonifier aufgeschlossen. Die Zelltrümmer wurden durch hochtourige Zentrifugation bei 15000 g 15 Minuten lang abzentrifugiert und der resultierende Überstand für die Aktivitätsbestimmungen benutzt. Der Aspartatkinase Test und der Dihydrodipicolinatsynthase Test wurden, wie bei Cremer et al. (a. a. 0.) beschrieben, durchgeführt. Die zugehörigen Proteinkonzentrationen wurden nach der Methode von Lowry et al. ermittelt (Lowry et al., J Biol Chem 193 (1951) 265). Der Gehalt an Enzym, und die Hemmbarkeit der Kinase sind in Tabelle 1 dargestellt. Es ist ersichtlich, daß hohe Enzymaktivitäten der entscheidenden Enzyme der Lysinbiosynthesequenz resultieren, und die Aspartatkinase keiner feedback Inhibierung durch L-Lysin plus L-threonin unterliegt.

Beispiel 2:

Ausscheidung von L-Lysin mit Plasmiden die lysC und dapA in Kombination enthalten.

Die in Beispiel 4 beschriebenen rekombinanten Stämme, sowie die Kontrollen wurden auf dem in Beispiel 4 beschriebenen Komplexmedium angezogen. Nach 17 Stunden Inkubation bei 30 °C wurden die Zellen geerntet, in 0,9% NaCl resuspendiert, und mit einer Animpfdichte von 1 auf das Fermentationsmedium folgender Zusammensetzung übertragen: 20 g/l $(NH_4)_2SO_4$, 0,5 g/l $KH_2PO_4$, 0,5 g/l $K_2HPO_4$, 0,25 g/l $MgSO_4 x7H_2O$, 10 mg/l $FeSO_4 x7H_2O$, 10 mg/l $MnSO_4 x7H_2O$, 1 mg/l $ZnSO_4 x7H_2O$, 0,2 mg/l $CuSO_4$, 0,02 mg/l $NiCl_2 x6H_2O$, 0,2 mg Biotin. Nach dem Autoklavieren wurden 2,4 g Glukose und 1,2 g $CaCO_3$ zu 60 ml Portionen zugefügt. Die Kolben wurden bei 30 °C auf einem Schüttler bei 140 Umdrehungen pro Minute inkubiert. nach 72 Stunden wurden Proben des Kulturfiltrats entnommen, abzentrifugiert, und im klaren Überstand die akkumulierte Menge an gebildetem L-Lysin bestimmt mittels eines Aminosäureanalysators bestimmt. Tabelle 2 zeigt die erhaltenen Werte.

Tabelle 1: Expression der Aspartatkinase und der Dihydrodipicolinatsynthase, sowie Restaktivität der Aspartatkinase bei Anwesenheit von je 10 mM L-Lysin und L-Threonin.

| | Sp. Aktivität (U/mg) | | |
|---|---|---|---|
| Stamm/Plasmid | Dihydrodipicolinat-synthase | Aspartat-kinase | Aspartatkinase + L-Lys, L-Thr |
| 13032 | 0,20 | 0,012 | 0,002 |
| 13032/pJC30 | 0,20 | 0,220 | 0,200 |
| 13032/pJC23 | 3,10 | 0,012 | 0,001 |
| 13032/pJC50 | 1,95 | 0,180 | 0,210 |
| DG52-5 | 0,21 | 0,014 | 0,003 |
| DG52-5/pJC30 | 0,21 | 0,240 | 0,200 |
| DG52-5/pJC23 | 1,85 | 0,015 | 0,003 |
| DG52-5/pJC50 | 2,05 | 0,210 | 0,210 |

Tabelle 2: Steigerung der Lysinausscheidung durch Kombination zweier

Enzymaktivitäten in Corynebacterium glutamicum.

| Stamm/Plasmid | relevantes Enzym | Akkumuliertes L-Lysin (mM) |
|---|---|---|
| 13032 | | 0 |
| 13032/pJC30 | Kinase | 38 |
| 13032/pJC23 | Synthase | 12 |
| 13032/pJC50 | Kinase plus Synthase | 50 |
| DG52-5 | | 43 |
| DG52-5/pJC30 | Kinase | 48 |
| DG52-5/pJC23 | Synthase | 52 |
| DG52-5/pJC50 | Kinase plus Synthase | 71 |

Es zeigen:

Fig. 1 : Konstruktion eines lys C$_D$ und dap A tragenden Pendelvektors pJC 50 (Bg = Bgl II, C = Cla I, D = Dra I, E = Eco RI, N = Nae I, P = Pst I, Pv = PVU II, S = Sal I, Sm = Sma I, Sp = Sph I, Xh = Xho I)

Fig. 2 : Deletionsanalyse des dap A/B Clusters aus C. glutamicum (B = Bam HI, Bg = Bgl II, C = Cla I, H = Hind III, P = Pst I, Pv = Pvu II, S = Sal I, Sp = Sph I)

Fig. 3 Deletionsanalyse des Lys C/asd Operons in Corynebacterium glutamicum (B Bam HI, D Dra I, E EcoR I, N Nae I, P Pst I, S Sal I, X Xho I, Xb XBa I)

**Patentansprüche**

1. Verfahren zur Herstellung von Aminosäure, insbesondere von L-Lysin, durch Züchten eines Mikroorganismus der Gattung Corynebacterium oder Brevibacterium, der eine rekombinante DNA aus Vektor-DNA und für die Produktion von Proteinen mit dapA-Aktivität kodierende DNA-Sequenz enthält, in einem Nährmedium und Gewinnung der Aminosäure aus dem Kulturmedium,
   **dadurch gekennzeichnet,**
   daß die rekombinante DNA zusätzlich eine für die Produktion von Proteinen mit lysC-Aktivität kodierende DNA-Sequenz enhält.

2. Verfahren nach Anspruch 1,
   **dadurch gekennzeichnet,**
   daß die kodierenden DNA-Sequenzen aus Stämmen der Gattung Corynebacterium oder Brevibacterium gewonnen worden sind.

3. Verfahren nach Anspruch 2,
   **dadurch gekennzeichnet,**
   daß die kodierenden DNA-Sequenzen aus L-Lysin produzierenden Stämmen der Gattung Corynebacterium oder Brevibacterium gewonnen worden sind.

**4.** Verfahren nach Anspruch 2,
**dadurch gekennzeichnet,**
daß die DNA-Sequenzen aus einer durch Mutagenese von Corynebacterium glutamicum ATCC 13032 erzielten Mutante mit verminderter Feed back Hemmung der Aspartatkinase erhalten worden sind.

**5.** Verfahren nach Anspruch 4,
**dadurch gekennzeichnet,**
daß die DNA-Sequenzen aus dem Stamm DSM 5714 erhalten worden sind.

**6.** Verfahren nach Anspruch 1,
**dadurch gekennzeichnet,**
daß als transformierter Mikroorganismus der Stamm Corynebacterium glutamicum DSM 5715 eingesetzt wird.

**7.** In Corynebacterium oder Brevibacterium replizierbare rekombinante DNA aus Vektor-DNA und für die Produktion von Proteinen mit dapA-Aktivität und lysC-Aktivität kodierenden DNA-Sequenzen.

**8.** Rekombinante DNA nach Anspruch 7,
**gekennzeichnet durch**
eine Vektor-DNA aus den Plasmiden pZ1, pJC1, pAJ1, pAJ3, pAJ4 und pAJ6 sowie pCG1 oder pCE5.

**9.** Mit rekombinanter DNA nach Anspruch 7 oder 8 tranformierte, L-Lysin produzierende Mikroorganismen der Gattung Corynebacterium oder Brevibacterium.

**Claims**

**1.** Process for preparing amino acid, in particular L-lysine, by cultivating a microorganism of the genus Corynebacterium or Brevibacterium, which harbours a recombinant DNA consisting of vector DNA and of a DNA sequence coding for the production of proteins having dapA activity, in a nutrient medium and isolating the amino acid from the culture medium, characterized in that the recombinant DNA additionally contains a DNA sequence coding for the production of proteins having lysc activity.

**2.** Process according to Claim 1, characterized in that the coding DNA sequences have been isolated from strains of the genus Corynebacterium or Brevibacterium.

**3.** Process according to Claim 2, characterized in that the coding DNA sequences have been isolated from strains of the genus Corynebacterium or Brevibacterium which produce L-lysine.

**4.** Process according to Claim 2, characterized in that the DNA sequences have been obtained from a mutant which has decreased feed-back inhibition of aspartate kinase and which was arrived at by mutagenizing Corynebacterium glutamicum ATCC 13032.

**5.** Process according to Claim 4, characterized in that the DNA sequences have been obtained from the strain DSM 5714.

**6.** Process according to Claim 1, characterized in that the strain Corynebacterium glutamicum DSM 5715 is employed as the transformed microorganism.

**7.** Recombinant DNA, consisting of a vector DNA and of DNA sequences coding for the production of proteins having dapA activity and lysC activity, which is capable of being replicated in Corynebacterium or Brevibacterium.

**8.** Recombinant DNA according to Claim 7, characterized by a vector DNA consisting of the plasmids pZ1, pJC1, pAJ1, pAJ3, pAJ4 and pAJ6, as well as pCG1 or pCE5.

**9.** L-lysine-producing microorganisms of the genus Corynebacterium or Brevibacterium which are transformed with recombinant DNA according to Claim 7 or 8.

7

**Revendications**

1. Procédé de préparation d'un amino-acide, en particulier de L-lysine, par mise en culture d'un micro-organisme de l'espèce *Corynebacterium* ou *Brevibacterium*, qui contient un ADN recombinant provenant d'un ADN vecteur et une séquence d'ADN codant pour la production de protéines ayant une activité de dapA, dans un milieu nutritif, et obtention de l'amino-acide à partir du milieu de culture, caractérisé en ce que l'ADN recombinant contient en outre une séquence d'ADN codant pour la production de protéines ayant une activité de lysC.

2. Procédé selon la revendication 1, caractérisé en ce qu'on obtient les séquences d'ADN codant à partir de souches de l'espèce *Corynebacterium* ou *Brevibacterium*.

3. Procédé selon la revendication 2, caractérisé en ce qu'or obtient les séquences d'ADN codant à partir de souches de l'espèce *Corynebacterium* ou *Brevibacterium* produisant la L-lysine.

4. Procédé selon la revendication 2, caractérisé en ce qu'on obtient les séquences d'ADN à partir d'un mutant obtenu par mutagénèse de *Corynebacterium glutamicum* ATCC 13032 avec une rétro-inhibition diminuée de l'aspartate-kinase.

5. Procédé selon la revendication 4, caractérisé en ce qu'on obtient les séquences d'ADN à partir de la souche DSM 5714.

6. Procédé selon la revendication 1, caractérisé en ce qu'on utilise, comme micro-organisme transformé, la souche *Corynebacterium glutamicum* DSM 5715.

7. ADN recombinant provenant d'un ADN vecteur, pouvant être reproduit dans l'espèce *Corynebacterium* ou *Brevibacterium*, et séquences d'ADN codant pour la production de protéines ayant une activité de dapA et une activité de lysC.

8. ADN recombinant selon la revendication 7, caractérisé par un ADN vecteur provenant des plasmides pZ1, pJC1, pAJ1, pAJ3, pAJ4 et pAJ6, ainsi que pCG1 ou pCE5.

9. Micro-organismes de l'espèce *Corynebacterium* ou *Brevibacterium*, produisant la L-lysine et transformés avec un ADN recombinant selon la revendication 7 ou 8.

Fig. 1

C. glu. dap A/B CLUSTER

Fig. 2

EP 0 435 132 B1

## C. glu. Lys C/asd OPERON

Komplementation Asp.kin. ASA DH
RASA 51 spez.Akt. spez.Akt.
asd − U/mg U/mg

| | Komplementation RASA 51 asd − | Asp.kin. spez.Akt. U/mg | ASA DH spez.Akt. U/mg |
|---|---|---|---|
| pJC 30 | + | 0,094 | 0,159 |
| pJC 31 | + | 0,024 | 0,229 |
| pJC 32 | − | − | 0,019 |
| pJC 33 | − | 0,102 | − |
| pJC 34 | + | − | 0,019 |
| 13032 | | 0,014 | 0,019 |

Fig. 3